(19) Europäisches Patentamt
European Patent Office
Office européen des brevets

(11) **EP 1 545 495 B1**

(12) **EUROPEAN PATENT SPECIFICATION**

(45) Date of publication and mention of the grant of the patent:
**04.04.2012 Bulletin 2012/14**

(21) Application number: **03741600.5**

(22) Date of filing: **21.07.2003**

(51) Int Cl.:
*A61K 31/245* [(2006.01)]   *A61K 31/352* [(2006.01)]
*A61K 31/337* [(2006.01)]   *A61K 31/704* [(2006.01)]
*A61K 45/06* [(2006.01)]   *A61P 35/00* [(2006.01)]

(86) International application number:
**PCT/KR2003/001441**

(87) International publication number:
**WO 2004/009073 (29.01.2004 Gazette 2004/05)**

(54) **P-GLYCOPROTEIN INHIBITOR COMPRISING OCTILONIUM BROMIDE AS AN EFFECTIVE INGREDIENT**

P-GLYCOPROTEIN-HEMMER MIT OCTILONIUMBROMID ALS WIRKSTOFF

INHIBITEUR DE P-GLYCOPROTEINE CONTENANT DU BROMURE D'OCTYLONIUM COMME INGREDIENT ACTIF

(84) Designated Contracting States:
**AT BE BG CH CY CZ DE DK EE ES FI FR GB GR HU IE IT LI LU MC NL PT RO SE SI SK TR**

(30) Priority: **20.07.2002 KR 2002042794**

(43) Date of publication of application:
**29.06.2005 Bulletin 2005/26**

(73) Proprietor: **DAEHWA PHARM. CO., LTD.**
**Gangwon-do (KR)**

(72) Inventors:
• **CHUNG, Hesson**
  **Incheon 402-715 (KR)**
• **JEONG, Seo-Young,**
  **Munchonmaeul Life Apt.**
  **Gyeonggi Do 411-747 (KR)**
• **KWON, Ick-Chan**
  **Seoul 139-230 (KR)**
• **PARK, Yeong-Taek**
  **Ansan, Gyeonggi-Do 425-735 (KR)**
• **LEE, In-Hyun**
  **Seoul 156-011 (KR)**
• **YUK, Soon-Hong**
  **Daejeon 305-755 (KR)**

(74) Representative: **Mai, Dörr, Besier**
**Patentanwälte**
**Steuerberater/Wirtschaftsprüfer**
**John-F.-Kennedy-Straße 4**
**65189 Wiesbaden (DE)**

(56) References cited:
EP-A1- 0 787 716   WO-A-02/13815
WO-A-98/17648   WO-A-99/17757
WO-A2-03/082199   US-A- 4 978 772

• G. ECKER AND OTHERS: "The importance of a nitrogen atom in modulators of multidrug resistance" MOLECULAR PHARMACOLOGY, vol. 56, 1999, pages 791-796, XP002410975
• GILLES KLOPMAN AND OTHERS: "Quantitative structure-activity relationship of multidrug resistance reversal agents" MOLECULAR PHARMACOLOGY, vol. 52, 1997, pages 323-334, XP002410976
• POYNARD ET AL.: 'Meta-analysis of smooth muscle relaxants in the treatment of irritable bowel syndrome' ALIMENTARY PHARMACOLOGY AND THERAPEUTICS vol. 15, no. 3, 2001, pages 355 - 361, XP003010305
• SANTICIOLI ET AL.: 'Antimuscarinic, calcium channel blocker and tachykinin NK2 receptor antagonist actions of otilonium bromide in the circular muscle of guinea-pig colon' NAUNYN-SCHMIEDEBERG'S ARCHIVES OF PHARMACOLOGY vol. 359, no. 5, 1999, pages 420 - 427, XP003010306
• EVANGELISTA ET AL.: 'RECEPTOR BINDING PROFILE OF OTILINIUM BROMIDE' PHARMACOLOGICAL RESEARCH vol. 38, no. 2, August 1998, pages 111 - 117, XP003010307

EP 1 545 495 B1

- **GANDIA ET AL.: 'Otilonium: a potent blocker of meuronal nicotinic ACh receptors in bovine chromaffin cells' BRITISH JOURNAL OF PHARMACOLOGY vol. 117, no. 3, 1996, pages 463 - 470, XP008078254**

Remarks:

The file contains technical information submitted after the application was filed and not included in this specification

## Description

**[TECHNICAL FIELD]**

The present invention relates to the use of octylonium bromide to increase cellular uptake of paclitaxel, rhodamine 123 or doxorubicin.

**[BACKGROUND ART]**

Octylonium bromide is an oral medication currently prescribed for the treatment of gastralgia and the irritable bowel syndrome. Octylonium bromide has an antispasmodic activity by loosening the contracted muscle in the stomach and the intestine resulted from hypersensitive reactions. Also, octylonium bromide is known as a medication of irritable bowel syndrome by controlling the motility and tension of the intestine. Since octylonium bromide is not absorbed into the intestine and acts mainly on the smooth muscle in the gastrointestinal tract, it does not have systemic side effects of anti-choline drugs including drowsiness and polydipsia. Octylonium bromide is also known as otilinium bromide or otilonium bromide.

P-Glycoprotein (pGP) is a product of multidrug resistance gene (MDR gene), and exists in the cell membrane to prevent the entrance of many toxic materials into the cytosol. P-Glycoprotein expels various absorbed toxic materials, especially anticancer drugs, out of the cell. Cancer cells that express p-glycoprotein do not respond well to the anticancer drug treatment and, the drug resistance increases with repeated dose of anticancer drugs.

P-Glycoprotein is known to distribute in the blood-brain barrier (BBB) and mucous cells in the intestine. To overcome the anticancer drug resistance and to make the anticancer drug or other physiologically active compounds to be absorbed upon oral administration, therefore, it is essential to develop drugs that can inhibit the activity of p-glycoprotein. Many p-glycoprotein inhibitors are known up to date including cinchonin, calcium channel blockers such as verapamil and dihydropyridines (for instance nifedipine, nicardipine and nitrendipine), calmodulin antagonists such as trifluoroperazine, Vinca alkaloids such as vincristine and vinblastine, and immunosuppressants such as cyclosporine A. Among them verapamil used for irregular heartbeats or chest pain (angina) can cause nausea and gastroenteric disorder. Nifedipine, used to treat high blood pressure, can cause side-effects such as low blood pressure, dizziness, flushing (feeling of warmth), constipation and nausia. Vincristine and Vinblastine can also cause severe side-effects. Cyclospone A, one of the most potent p-glycoprotein inhibitors, can cause harmful effects on the immune system since it is an immunosuppressant.

To overcome these problems, many p-glycoprotein inhibitors with lower side-effects and higher activity are being developed. A cyclosporin analog, SDZ PSC833, which does not lower the immune function was developed (Norvatis). Indane derivative that can inhibit p-glycoprotein was also synthesized by Dr. Yoo in Korea Research Institute of Chemical Technology.

WO98/17648 describes the use of structurally closely related compounds as multi-drug resistance modulators and P-gp inhibitors (claims 1, 13 and 14) and their use in combination with anti-cancer drugs. The claimed compounds are used to potentiate the cytotoxicity of an agent which is cytotoxic to a tumour cell and to enhance the absorption, distribution or metabolism of said drug by inhibiting P-gp and thus overcoming multi-drug resistance (p. 30, par. 2-3). The cytotoxic drug that is used may be doxorubicin or taxol. The claimed compounds are structurally closely related to octylonium bromide but differ from octylonium bromide in that they all have an NO2 group attached to the first benzene ring. Moreover the compounds are lacking a quaternary amine and an ester group, which are essential elements of octylonium bromide and have an additional ring system.

While searching for p-glycoprotein inhibitors, the present inventors have discovered that octylonium bromide, a calcium channel blocker, can help the absorption of drugs that are pumped out of the intestinal cells by p-glycoprotein. Octylonium bromide as the absorption enhancer in the present invention has a broader applicability and less toxicity than the absorption enhancer described in US 5,968,972. When administered orally, only 5 % of octylonium bromide is absorbed systematically, and the rest 95 % remains in the gastrointestinal tract. Since the local concentration in the intestine is higher, octylonium bromide can increase the oral bioavailability of the drugs effectively. The unabsorbed 95 % is discharged, and therefore minimizing the systemic toxicity. Also, the concentration of the anticancer drug in the blood increases with an increasing dose of octylonium bromide.

< Summary of the Invention >

The object of the present invention is to provide the use of octylonium bromide as an inhibitor of p-glycoprotein, and thus as an absorption enhancer of drugs that have a low oral bioavailability by co-administration, namely paclitaxel, doxorubicine and rhodamine 123.

Another object of the present invention is to provide the use of octylonium bromide as an absorption enhancer of said drugs that have a low oral bioavailability by co-administration.

**[DETAILED DESCRIPTION OF THE INVENTION]**

Also disclosed is the use of octylonium bromide as absorption enhancer of anticancer drugs that exist in the mucous cells in the intestine.

In the present invention, octylonium bromide, as an effective ingredient of a p-glycoprotein inhibitor, is effective at the dose of 0.01 mg/kg to 1 g/kg of body weight. When the dose of octylonium bromide is lower than 0.01 mg/kg, it cannot inhibit p-glycoprotein. When the dose exceeds 1 g/kg, however, gastroenteric disorder can be caused.

Also, the present invention provides a slow release formula of octylonium bromide that can provide the p-glycoprotein inhibition activity for the duration of 12 hours. The slow release formula is prepared by preparing uniform sized seed granules with regular size, by coating the granules with octylonium bromide and by coating the exterior layer with polymer that can control the release rate of drug.

Octylonium bromide according to the present invention can be administered together with or 30 minutes prior to the administration of the drugs paclitaxel, doxorubicin and rhodamine 123.

In order to increase the availability of anticancer drugs, octylonium bromide can be co-administered with the claimed anticancer drugs via intravenous injection, intramuscular injection, intratumoral injection, subcutaneous injection, oral administration, intravesical administration or intraperitoneal administration. Among them, oral administration is the most preferable. Octylonium bromide can be administered as a tablet or capsule.

The drugs that have enhanced oral bioavailability when administered with octylonium bromide are the anticancer drugs doxorubicin, and paclitaxel, and the fluorescent chemical rhodamine 123.

Above octylonium bromide can increase the absorption of drugs encapsulated in the oily solution solubilizing insoluble drugs. The above oily solution can include at least one monoglyceride, at least one oil and at least one emulsifier

The above monoglycerides are selected from a group consisting of one or more saturated or an unsaturated monoglycerides having 10 ~ 22 carbon atoms in the hydrocarbon chain. Monoglycerides is selected preferably from a group consisting of monoolein, monopalmitolein, monomyristolein, monoelaidin and monoerucin or from a group consisting of the mixture of monoglycerides semi-synthesized from triglycerides of vegetable or animal oil, and more preferably monoolein.

The above oil is selected preferably from a group consisting of triglycerides, iodinated oil, vegetable oil and animal oil.

The above triglycerides are selected from a group consisting of one or more saturated or unsaturated triglycerides having 2 ~ 20 carbon atoms in the hydrocarbon chain. For instance, triacetin, tributyrin, tricaproin, tricaprylin, tricaprin or triolein can be used.

The above iodized oils include iodized poppy seed oil such as Lipiodol, Ethiodol and iodized soybean oil.

The above vegetable oils include soybean oil, cottonseed oil, olive oil, poppyseed oil, linseed oil or sesame oil.

The above animal oils include squalane or squalene.

The emulsifier is preferred to select from the group consisting of a phospholipid, a non-ionic surfactant, an anionic surfactant, a cationic surfactant, and bile acid.

The phospholipid is preferred to select from the group consisting of a phosphatidylcholine (PC) and its derivative, a phosphatidylethanolamine (PE) and its derivative, a phosphatidylserine (PS) and its derivative and a polymeric lipid wherein a hydrophilic polymer is conjugated to the lipid headgroup.

The non-ionic surfactant is selected from the group consisting of a poloxamer (also known as Pluronic: polyoxyethylene-polyoxypropylene copolymer), a sorbitan ester (Span), a polyoxyethylene sorbitan (Tween) and a polyoxyethylene ether (Brij).

The anionic surfactant is selected from the group consisting of a phosphatidylserine (PS) and its derivative, a phosphatidic acid (PA) and its derivative or sodium dodecyl sulfate (SDS).

The cationic surfactant is selected from the group consisting of 1,2-dioleyl-3-trimethylammonium propane (DOTAP), dimethyldioctadecylammonium bromide (DDAB), N-[1-(1,2-dioleyloxy)propyl]-N,N,N-trimethylammonium chloride (DOTMA), 1,2-dioleyl-3-ethylphosphocholine (DOEPC) and 3β-[N-[(N',N'-dimethylamino)ethan]carbamoyl]cholesterol (DC-Chol).

The bile acid is selected from the group consisting of cholic acid, its salt and derivatives; deoxycholic acid, its salt and derivatives; chenocholic acid, its salt and derivatives; and lithocholic acid, its salt and derivatives.

Other additives can be added to the above composition to be within 5% by weight (with respect to the total weight of the composition). For instance, the composition can further comprise alcohol, polyol or Cremophor to improve the solubility of the drugs, tocopherol or tocopherol acetate to prevent oxidation, and fatty acid, fatty acid ester or fatty acid alcohol to increase drug absorption.

## BRIEF DESCRIPTION OF THE DRAWINGS

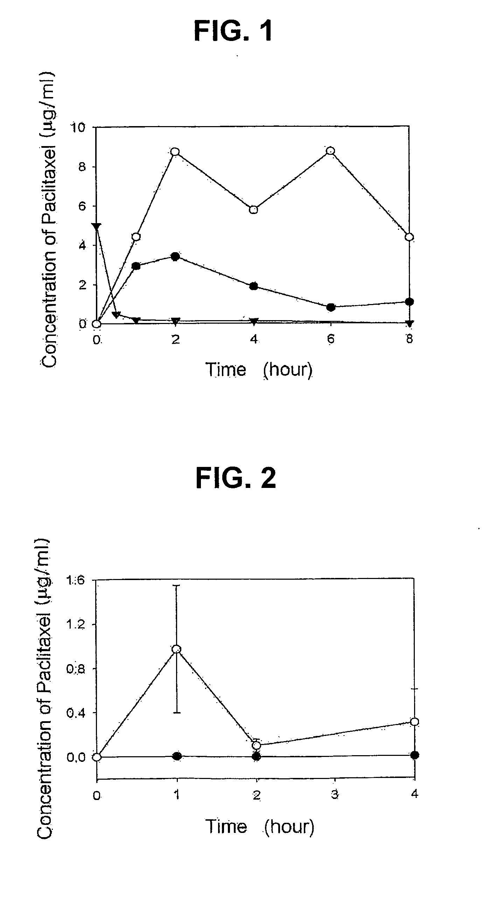

Figure 1 is a graph showing the total concentration of paclitaxel and their metabolites in blood after oral administration of Taxol® of Bristol-Myers Squibb Company and octylonium bromide as an absorption enhancer. The quantitative analysis was performed by ELISA.

- ● - ; a group orally administered with Taxol® of Bristol-Myers Squibb Company (1 mg paclitaxel),
- ○ - ; a group orally administered with Taxol® of Bristol-Myers Squibb Company (1 mg paclitaxel) 30 minutes after administering orally 2 mg of octylonium bromide, and
- ▲ - ; a group intravenously administered with Taxol® of Bristol-Myers Squibb Company (10 μg paclitaxel).

Figure 2 is a graph showing the total concentration of paclitaxel and their metabolites in blood after oral administration of tricaprylin emulsion encapsulating paclitaxel and octylonium bromide as an absorption enhancer. The quantitative analysis was performed by ELISA.

- ● - ; a group orally administered with tricaprylin emulsion encapsulating paclitaxel (1 mg paclitaxel), and
- ○ - ; a group orally administered with tricaprylin emulsion encapsulating paclitaxel (1 mg paclitaxel) 30 minutes after administering orally 2 mg of octylonium bromide.

Figure 3 is a graph showing the total concentration of paclitaxel and their metabolites in blood after oral administration of oily solution containing paclitaxel and octylonium bromide as an absorption enhancer. The quantitative analysis was performed by ELISA.

- ● - ; a group orally administered with oily solution containing paclitaxel (1 mg paclitaxel), and
- ○ - ; a group orally administered with oily solution containing paclitaxel (1 mg paclitaxel) 30 minutes after administering orally 2 mg of octylonium bromide.

Figure 4 is a graph showing the total concentration of paclitaxel and their metabolites in blood after oral administration of oily solution containing paclitaxel and different amounts of octylonium bromide as an absorption enhancer. The quantitative analysis was performed by ELISA.

- ● - ; a group orally administered with oily solution containing paclitaxel (1 mg paclitaxel),
- ○ - ; a group orally administered with oily solution containing paclitaxel (1 mg paclitaxel) and 0.5 mg of octylonium bromide,
- ▲ - ; a group orally administered with oily solution containing paclitaxel (1 mg paclitaxel) and 2 mg of octylonium bromide, and
- △ - ; a group orally administered with oily solution containing paclitaxel (1 mg paclitaxel) and 4 mg of octylonium bromide.

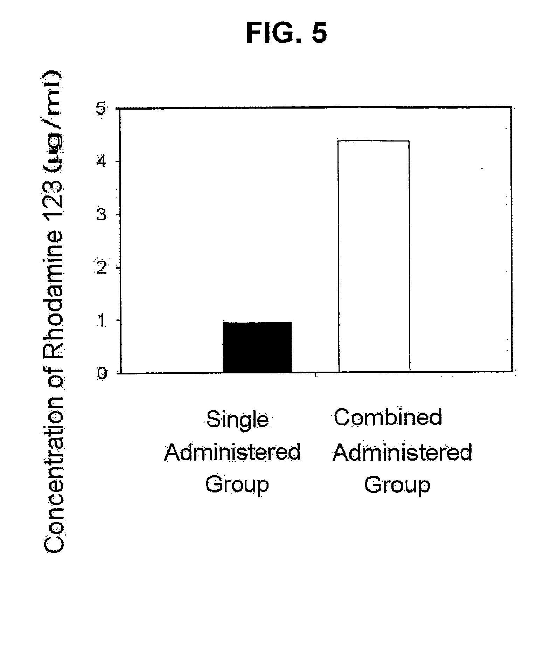

Figure 5 is a graph showing the concentration of absorbed rhodamine 123 into the intestine depending on the concentration of octylonium bromide by using everted sac.

□; a group treated with 10 μg/ml of rhodamine 123, and
■; a group treated with 10 μg/ml of rhodamine 123 and 200 μg/ml of octylonium bromide.

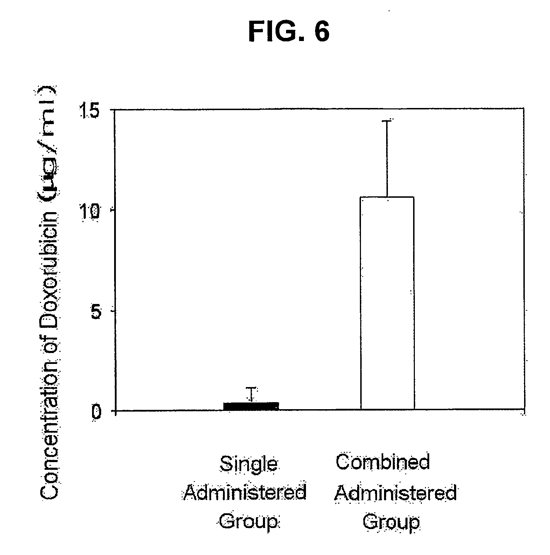

Figure 6 is a graph showing the concentration of absorbed doxorubicin into the intestine depending on the concentration of Octylonium bromide by using everted sac.

□; a group treated with 50 μg/ml of doxorubicin, and
■; a group treated with 50 μg/ml of doxorubicin and 200 μg/ml of octylonium bromide.

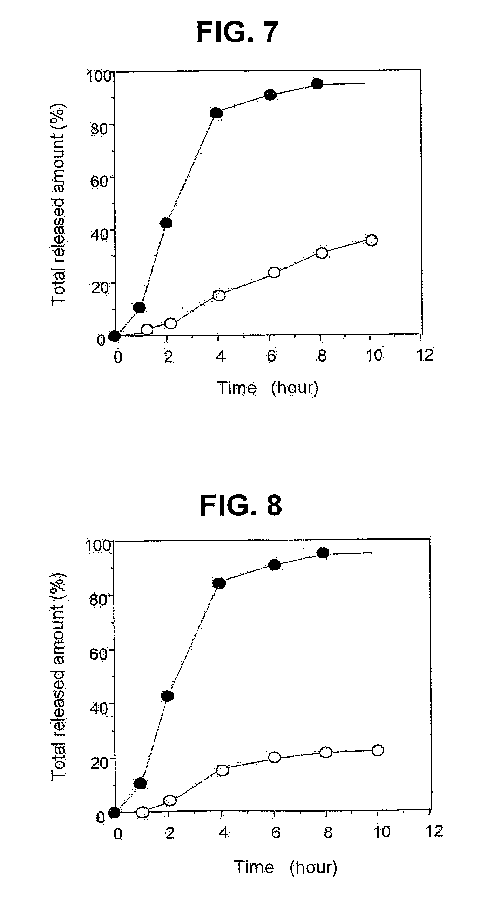

Figure 7 is a graph showing the release rate of octylonium bromide from the sustained release granules containing octylonium bromide prepared by external coating with compositions containing Eudragit RS 100.

- ● - ; granules with an external layer coating by a composition containing 10 % of Eudragit RS 100, and
- ○ - ; granules with an external layer coating by a composition containing 20 % of Eudragit RS 100.

Figure 8 is a graph showing the release rate of octylonium bromide from the sustained release particle containing octylonium bromide prepared by external coating with compositions containing Eudragit NE 30 D.

- ● - ; granules with an external layer coating by a composition containing 10 % of Eudragit NE 30 D, and
- ○ - ; granules with an external layer coating by a composition containing 20 % of Eudragit NE 30 D.

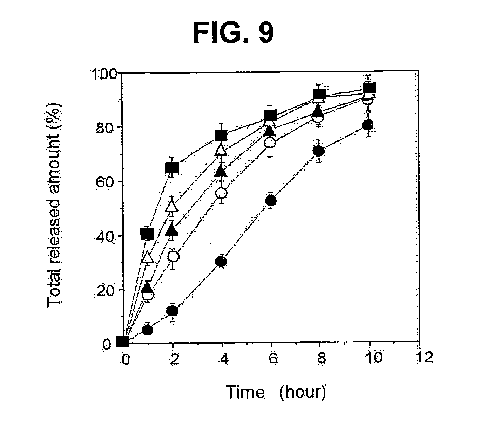

Figure 9 is a graph showing the release rate of octylonium bromide from the sustained release particle containing octylonium bromide prepared by external coating with compositions containing 10 % of Eudragit RS 100 and different amounts of HPMC.

- ● granules with an external layer coating by a composition additionally containing 10 % of HPMC,
- ○ - ; granules with an external layer coating by a composition additionally containing 20 % of HPMC,
- ▲ - ; granules with an external layer coating by a composition additionally containing 30 % of HPMC,
- △ - ; granules with an external layer coating by a composition additionally containing 40 % of HPMC, and
- ■ - ; granules with an external layer coating by a composition additionally containing 50 % of HPMC.

**[Best Mode for Carrying Out the Invention]**

The following examples show that absorption of the claimed drugs, whose absorption is inhibited by p-glycoprotein, is enhanced by oral administration of octylonium bromide. This invention is explained in more detail based on the following Examples

**Example 1. Oral administration of Taxol®**

① Oral administration

Taxol® was administered into Balb/C mouse (6 ~ 7 weeks old, female) fasted for 4 hours previously by using a gastric sonde. One hundred and sixty seven microliters of Taxol® of Bristol-Myers Squibb Company was mixed with 0.5 ml water. As a single administered group, the above solution containing paclitaxel (corresponding to 1 mg paclitaxel per mouse) was administered orally. Another group of mice, a co-administered group, was orally administered with 2 mg octylonium bromide dissolved in 200 $\mu$l of phosphate buffered saline (PBS) and, after 30 minutes, 167 $\mu$l Taxol® of Bristol-Myers Squibb Company mixed with 0.5 ml water. Concentration of paclitaxel in blood was determined 1, 2, 4, 6 and 8 hours after the oral administration by collecting blood from the eyes.

As a control group, Taxol® of Bristol-Myers Squibb Company was administered intravenously into Balb/C mouse (6 ~ 7 weeks old, female). The concentration of paclitaxel in blood was determined up to 8 hours after the administration. After mixing 0.1 ml of Taxol® with 5.9 ml of water, 0.1 ml of the mixture corresponding to 10 $\mu$g of Taxol® was administered by bolus injection through tail vein. Concentration of paclitaxel in blood was determined 0.5, 1, 2, 4 and 8 hours after the oral administration by collecting blood from the eyes.

② Determination of total concentration of paclitaxel and its metabolites in blood (ELISA method)

The total concentration of paclitaxel and its metabolites in blood was determined by using Anti-taxane monoclonal kit (Model number 8A10) of Hawaii Biotech Company. Paclitaxel is known to be converted to 6-$\alpha$-hydroxypaclitaxel and 3'-p-hydroxypaclitaxel by CYP2C8 and CYP3A4, respectively. Various metabolites including the primary metabolites of paclitaxel exist in the blood. Anti-taxane monoclonal kit enables us to determine the concentration of paclitaxel and all of the metabolites containing taxane ring (Grothaus, G.P., Bignami, G.S., O'Malley, S., Harada, K.E., Byrnes, J.B., Waller, D.F., Raybould, T.J.G., Mcguire, M.T. and Alvaro, B., Taxane-specific monoclonal antibodies: measurement of taxol, baccatin III, and 'total taxanes' in Taxus brevifolia extracts by enzyme immunoassay. J. Nat. Prod. 58, pp. 1003-1014, 1995).

The blood sample was serially diluted 4 times. Taxol-protein coating antigen (blue label) was diluted 100 times by phosphate buffered saline (PBS). After 100 $\mu$l of the diluted antigen solution was put into each well of the 96-well plate, the plate was incubated for 1 hour. After the plate was washed 4 times with TBST, it was blocked by adding PBS containing 1 % bovine serum albumin for 1 hour. After each well was washed continuously four times with TBST, 50 $\mu$l of the serially diluted samples were put into each well. After diluting HBC Taxol Standard (RED label) serially with PBST, 50 $\mu$l of the diluted standard solution was put into each well. Fifty microliters of the second antibody solution prepared by mixing 4.5 ml PBST and 50 $\mu$l of anti-taxane rabbit antibody (green label) was added in each well. After the wells were washed four times with TBST, 100 $\mu$l of secondary antibody solution diluted 1000 times with PBST was added and incubated for one hour. After washing the wells four times with TBST, 200 $\mu$l of pNPP solution at 1 mg/ml was added in each well. After incubating the plate for 1 hour at room temperature, the absorbance was measured by using ELISA reader at 414 nm and compared with that at 690 nm for quantitative analysis.

③ Results

The changes in the paclitaxel concentration in blood with time are shown in Figure 1. When the bioavailability of paclitaxel upon bolus injection was set to 100 %, the relative bioavailability upon oral administration of paclitaxel was calculated by the following formula.

$$\text{Bioavailability (\%)} = \frac{\text{AUCoral}}{\text{AUCiv}} \times \frac{\text{DOSEiv}}{\text{DOSEoral}} \times 100$$

Wherein, AUCoral and AUCiv represent area under the curve after oral and intravenous administration, respectively, and DOSEoral and DOSEiv represent the paclitaxel dose for the oral and intravenous administration, respectively. The bioavailability upon oral administration of Taxol® when compared to the bolus injection was 6.5 % whereas the bioavailability upon co-administration of Taxol® and octylonium bromide was 22.8 %. Co-administration of octylonium increased the bioavailability of Taxo®| by ca. 3.5 times.

**Example 2. Oral administration of tricaprylin emulsion encapsulating paclitaxel**

Viscous oily solution was prepared by mixing 1g tricaprylin, 0.2 g Tween 80 and 12 mg paclitaxel by warming at 40 ˚C and by sonicating in a bath type sonicator for complete solubilization. To the above oily solution, 4.85 ml of water was added and sonicated by using a probe type sonicator (High intensity ultrasonic processor, microprocessor control, 600-Watt model) for 2 min to prepare tricaprylin emulsion encapsulating paclitaxel. Paclitaxel precipitation was not observed under polarized light microscope, and phase separation was not observed either.

The tricaprylin emulsion encapsulating paclitaxel (1 mg paclitaxel per mouse) was administered into Balb/C mouse by using identical methods as in Example 1 (Group received paclitaxel alone). Another group was administered with a solution containing 2 mg octylonium bromide in 200 μl of phosphate buffer solution followed by 500 μl of the tricaprylin emulsion encapsulating paclitaxel (1 mg paclitaxel per mouse) after a 30 min interval (Group received paclitaxel and octylonium bromide). Blood was collected 1, 2 and 4 h after the administration of the compositions, the concentration of paclitaxel in the blood collected from the eyes was determined. The total concentration of paclitaxel and its metabolites in blood analyzed by ELISA is shown in Figure 2.

The bioavailability of the group received paclitaxel alone when compared to the bolus injection in Example 1 (1 μg paclitaxel per mouse) was 0.0 % indicating that paclitaxel was not absorbed at all. On the other hand, the bioavailability of the group received paclitaxel and octylonium bromide was 0.63 %.

**Example 3. Oral administration of oily solution encapsulating paclitaxel**

Viscous oily solution was prepared by mixing 1 g monoolein, 1 g tricaprylin and 0.4 g Tween 80 and by warming at 40 ˚C. Twenty four milligrams of paclitaxel was added into the oily solution and sonicated in a bath type sonicator for complete solubilization.

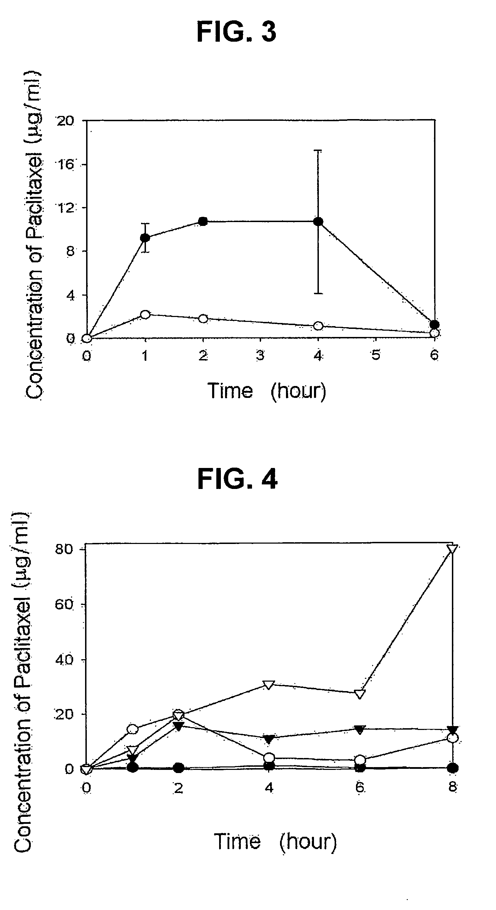

The oily solution encapsulating paclitaxel (1 mg paclitaxel per mouse) was administered into Balb/C mouse by using identical method as in Example 1 (Group received paclitaxel alone). Another group was administered with a solution containing 2 mg octylonium bromide in 200 μl of phosphate buffer solution followed by 100 μl of the oily solution encapsulating paclitaxel (1 mg paclitaxel per mouse) after a 30 min interval (Group received paclitaxel and octylonium bromide). Blood was collected 1, 2 and 4 h after the administration of the compositions, the concentration of paclitaxel in the blood collected from the eyes was determined. The total concentration of paclitaxel and its metabolites in blood analyzed by ELISA is shown in Figure 3.

The bioavailability of the group received paclitaxel alone when compared to the bolus injection in Example 1 (10 μg paclitaxel per mouse) was 1.0 % indicating that only a small amount of paclitaxel was absorbed. On the other hand, the bioavailability of the group received paclitaxel and octylonium bromide was 21.4 %.

**Example 4. Oral administration of oily solution encapsulating paclitaxel according to the dose of octylonium bromide**

One hundred microliters of the oily solution encapsulating paclitaxel prepared in Example 3 (1 mg paclitaxel per mouse) was administered into Balb/C mouse by using identical methods as in Example 1 (Group received paclitaxel only), together with various concentrations of octylonium bromide. Groups of mice were administered with solutions each containing 0.5, 2 and 4 mg octylonium bromide in 200 µl of phosphate buffer solution followed by 100 µl of the oily solution encapsulating paclitaxel (1 mg paclitaxel per mouse) after a 30 min interval (Groups received paclitaxel and octylonium bromide).

Blood was collected 1, 2 and 4 h after the administration of the compositions, the concentration of paclitaxel in the blood collected from the eyes was determined. The total concentration of paclitaxel and its metabolites in blood analyzed by ELISA is shown in Figure 4.

The bioavailability of the group received paclitaxel alone when compared to the bolus injection in Example 1 (10 µg paclitaxel per mouse) was 2.3 % indicating that only a small amount of paclitaxel was absorbed. On the other hand, the bioavailability of the group received paclitaxel and octylonium bromide increased with increasing dose of octylonium bromide as shown in Table 1.

**Table 1**

| Amount of orally administered octylonium bromide (mg) | Bioavailability (%) |
|---|---|
| 0 | 2.3 |
| 0.5 | 31.1 |
| 2 | 41.9 |
| 4 | 104.4 |

**Example 5. Ex Vivo Absorption experiment of rhodamine 123 by using everted sac model**

Oral absorption of rhodamine 123 is well-known to be inhibited by p-glycoprotein. Ileum portion of the intestine was taken out after sacrificing the SD rats. The ileum was cut into 2-cm tubes in length, and everted so as to expose the mucous intestinal tissue to the exterior of the tubes. After being tied both ends of the everted sac, the sac was immersed in 1 ml Krebs-Ringer buffer (KRB solution, pH 6.4) containing 10 µg/ml rhodamine 123. The immersed sac was stored in an oxygen supplied, 37 ˚C incubator for 1 hour (Group treated with rhodamine alone).

Another group of everted sacs was stored as described above with the exception that sacs were immersed in 1 ml Krebs-Ringer buffer containing 10 µg/ml rhodamine 123 and 10 µg/ml octylonium bromide (Group treated with rhodamine and octylonium bromide). After one hour of the incubation, the sacs were put in 1 ml Krebs-Ringer buffer. The sacs were homogenized and centrifuged. Supernatant was obtained to analyze the concentration of rhodamine 123 by Fluorimetry as shown in Figure 5. The amount of the absorbed rhodamine 123 in the group treated with rhodamine alone was 0.95 µg/g tissue whereas that in the group treated with rhodamine and octylonium bromide was 4.3 µg/g tissue. These correspond to 3 and 14.5 %, respectively, of the total applied amount. The results show that octylonium bromide helped increasing the amount of absorbed rhodamine by 4.5 folds.

**Example 6. Ex Vivo Absorption experiment of doxorubicin by using everted sac model**

Oral absorption of doxorubicin is well-known to be inhibited by p-glycoprotein. Everted sacs were used to perform the ex vivo absorption experiment by using the same method as in Example 5 excepting that 50 µg/ml doxorubicin was used instead of rhodamine 123.

The absorbed amount of the doxorubicin in the group treated with doxorubicin alone was 0.4 µg/g tissue whereas that in the group treated with doxorubicin and octylonium bromide was 11 µg/g tissue. These correspond to 0.12 and 3.3 %, respectively, of the total applied amount. The results show that octylonium bromide helped increasing the amount of absorbed doxorubicin by 28 folds.

**Example 7. Preparation of slow release granules of octylonium bromide**

1. Preparation of uniform sized granules

To prepare granules with uniform size, 355 - 500 µm size sugar particles were used as seeds and coated with a composition consisting of 300 g sugar, 100g HPMC 2910, 700 g corn starch, 20 g PEG 6000, 1050 g water, 950 g acetone and 1050 g ethanol by using a coating machine. The size of the prepared granules was 650 - 710 μm in diameter. The coating conditions are shown in Table 2.

**Table 2**

| Preheating : | 20 min |
|---|---|
| Inlet air temperature | 28 ˚C |
| Outlet air temperature | 23 ˚C |
| Inlet air flow setting | 30 |
| Spray: | 100 min |
| Spray nozzle diameter | 2.5 mm |
| Automizing air pressure | 1.8 ~ 2.3 bar |
| Outlet air temperature | 23 ˚C |
| Inlet air temperature | 32 ˚C |
| Inlet air flow setting | 30 |
| Type of collecting plate | D |
| Air flow rate | 20 |
| Spray pressure increase rate | 0.5 bar/5 flow rate |
| Flow rate increase rate | 5 flow rate /5 min |
| Temperature increase rate | 2˚C/5 min |
| Drying : | 30 min |
| Inlet air temperature | 40 ˚C |
| Outlet air temperature | 32 ˚C |
| Inlet air flow setting | 40 |

2. Coating of drug containing layer on the prepared granules

The prepared granules (200 g) were coated with a coating solution containing 26.7 g HPMC 2919, 200 g corn starch, 70 g octylonium bromide, 5.3 g PEG 6000, 700 g water, 350 g acetone and 500 g ethanol. The size of the prepared coated granules was 800 - 1000 μm in diameter and the yield was 90 %.

3. External layer coating to achieve slow release

To control the release rate of the drug, a composition containing polymetaacrylate compound, Eudragit RS or Eudragit NE was coated on the drug coated granules. The components and compositions of the Eudragit RS and Eudragit NE are shown in Table 3 and Table 4, respectively.

**Table 3. Composition of Eudragit RS 100 coating**

| Components | Weight (g) | |
|---|---|---|
| | Eudragit® 7%* | Eudragit® 14%* |
| particle | 800 | 800 |
| Eudragit® RS 100 | 56 | 112 |
| Methylene chloride | 428 | 856 |
| acetone | 408 | 816 |
| talc | 56 | 112 |
| Triethyl citrate | 8.4 | 16.8 |
| Total amount | 1,756.4 | 2,712.8 |

### Table 4. Composition of Eudragit NE 30 D coating

| Components | Weight (g) | |
|---|---|---|
| | Eudragit® 10%* | Eudragit® 20%* |
| particle | 800 | 800 |
| Eudragit® NE 30 D | 266 | 532 |
| water | 400 | 800 |
| talc | 56 | 112 |
| Triethyl citrate | 12 | 24 |
| Total amount | 1,756.4 | 2,268 |

**Example 8. Drug release experiment from the slow release granules of octylonium bromide coated with Eudragit RS 100**

The slow release granules of octylonium bromide coated with Eudragit RS 100 prepared in Example 7 was immersed in water to determine the amount of released octylonium bromide as a function of time (Figure 7). When the content of Eudragit was 7 %, 50 % of the drug was released in 4 hours. On the other hand, approximately 40 % of the drug was released in 10 hours when Eudragit content was 14 %. The results show that the release rate of the drug can be controlled.

**Example 9. Drug release experiment from the slow release granules of octylonium bromide coated with Eudragit NE 30 D**

The slow release granules of octylonium bromide coated with Eudragit NE prepared in Example 7 was immersed in water to determine the amount of released octylonium bromide as a function of time (Figure 8). When the content of Eudragit was 10 %, 50 % of the drug was released in 2 hours. On the other hand, approximately 20 % of the drug was released in 10 hours when Eudragit content was 20 %. The results show that the release rate of the drug can be controlled.

**Example 10. Drug release experiment from the slow release granules of octylonium bromide coated with Eudragit RS 100**

The granules were formulated by preparing granules by the same method as in above Example 7 and by coating the granules with the composition containing octylonium bromide. Slow release granules of octylonium bromide coated with Eudragit RS 100 prepared in Example 7 were immersed in water to determine the amount of released octylonium bromide as a function of time (Figure 7). In order to coat slow release external layer, the composition for external layer coating was prepared by adding 10, 20, 30, 40, and 50 % by weight of hydroxy propyl methyl cellulose 2910 (HPMC 2910) with respect to the weight of Eudragit RS 100 into the composition containing 7 % Eudragit RS 100. The prepared compositions were used for external layer coating. The results of the octylonium bromide release experiment are shown in Figure 9. The release rate became slower as the amount of HPMC increased.

**[Industrial Applicability]**

The present invention describes the use of octylonium bromide to lower the activity of p-glycoprotein. Octylonium bromide increased dramatically the bioavailability of paclitaxel when administered orally. Also, it is shown in the present invention that octylonium bromide inhibits the activity of p-glycoprotein by performing the rhodamine 123 absorption experiments. Therefore, octylonium bromide can be used to increase the bioavailability of drugs that are pumped out by p-glycoprotein.

**Claims**

1. Medicament containing octylonium bromide and a drug selected from the group consisting of paclitaxel, rhodamine 123 and doxorubicin.

2. The medicament according to Claim 1, wherein the dose of octylonium bromide is in the range between 0.01 mg/kg body weight and 1 g/kg body weight.

3. The medicament according to Claim 1, wherein octylonium bromide is formulated as a slow release formula to sustain the release of octylonium bromide for up to 12 hours.

4. The medicament according to Claim 3 formulated by preparing uniform sized granules as a seed, by coating the granules with a composition containing octylonium bromide and by coating polymer that can control the release rate of the drug to form an external layer.

5. The medicament according to Claim 1 for administration by intravenous injection, intramuscular injection, intratumoral injection, subcutaneous injection, oral administration, intravesical administration, or intraperitoneal administration.

6. The medicament according to Claim 5, wherein the type of the formulation is tablet or capsule.

7. Use of octylonium bromide and a drug selected from the group consisting of paclitaxel, rhodamine 123 and doxorubicin for the preparation of a medicament

**Patentansprüche**

1. Medikament, enthaltend Octyloniumbromid und einen Wirkstoff, ausgewählt aus der Gruppe bestehend aus Paclitaxel, Rhodamin 123 und Doxorubicin.

2. Medikament nach Anspruch 1, wobei die Dosierung von Octyloniumbromid in dem Bereich zwischen 0,01 mg/kg Körpergewicht und 1 g/kg Körpergewicht liegt.

3. Medikament nach Anspruch 1, wobei Octyloniumbromid als eine Depotformulierung formuliert ist, um die Freisetzung von Octyloniumbromid für bis zu 12 Stunden kontinuierlich aufrechtzuhalten.

4. Medikament nach Anspruch 3, formuliert durch Herstellung von Granulat mit einheitlicher Größe als Kern, durch Überziehen des Granulats mit einer Zusammensetzung, enthaltend Octyloniumbromid, und durch Überziehen mit einem Polymer, welches die Freisetzungsrate des Wirkstoffs kontrollieren kann, um eine äußere Schicht zu bilden.

5. Medikament nach Anspruch 1 zur Verabreichung durch intravenöse Injektion, intramuskuläre Injektion, intratumorale Injektion, subkutane Injektion, zur oralen Verabreichung, intravesikalen Verabreichung oder intraperitonealen Verabreichung.

6. Medikament nach Anspruch 5, wobei die Formulierungsart eine Tablette oder Kapsel ist.

7. Verwendung von Octyloniumbromid und einem Wirkstoff, ausgewählt aus der Gruppe bestehend aus Paclitaxel, Rhodamin 123 und Doxorubicin, für die Herstellung eines Medikaments.

**Revendications**

1. Médicament contenant du bromure d'octylonium et une drogue sélectée du groupe formé de paclitaxel, rhodamine 123 et doxorubicine.

2. Médicament selon la revendication 1, où la dose de bromure d'octylonium est dans la rangée entre 0,01 mg/kg poids corporel et 1 g/kg poids corporel.

3. Médicament selon la revendication 1, où le bromure d'octylonium est formulé comme une formule à libération lente pour soutenir la libération de bromure d'octylonium dans un temps de jusqu'à 12 heures.

4. Médicament selon la revendication 3 formulé par la préparation de granules de taille uniforme comme une graine, par le recouvrement des granules avec une composition contenant du bromure d'octylonium et par le polymère de revêtement qui peut contrôler la vitesse de libération de la drogue pour former une couche extérieure.

**5.** Médicament selon la revendication 1 pour administration par injection intraveineuse, injection intramusculaire, injection intratumorale, injection sous-cutanée, administration orale, administration intravésicale, ou administration intrapéritonéale.

**6.** Médicament selon la revendication 5, où le type de la formulation est la tablette ou la capsule.

**7.** Utilisation de bromure d'octylonium et d'une drogue sélectée du groupe formé de paclitaxel, rhodamine 123 et doxorubicine pour la préparation d'un médicament.

# FIG. 1

# FIG. 2

# FIG. 3

# FIG. 4

# FIG. 5

# FIG. 6

# FIG. 7

# FIG. 8

# FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9817648 A

- US 5968972 A

**Non-patent literature cited in the description**

- **Grothaus, G.P. ; Bignami, G.S. ; O'Malley, S. ; Harada, K.E. ; Byrnes, J.B. ; Waller, D.F. ; Raybould, T.J.G. ; Mcguire, M.T. ; Alvaro, B.** Taxane-specific monoclonal antibodies: measurement of taxol, baccatin III, and 'total taxanes' in Taxus brevifolia extracts by enzyme immunoassay. *J. Nat. Prod.,* 1995, vol. 58, 1003-1014